# EUROPEAN PATENT SPECIFICATION

(11) **EP 2 184 012 B1**
(45) Date of publication and mention of the grant of the patent: **19.06.2013**
(21) Application number: 08828783.4
(22) Date of filing: 29.08.2008
(51) Int. Cl.: A61B 5/15, A61B 5/151, A61B 5/157, A61B 17/34, A61M 1/02

(54) **ASSISTANCE DEVICE**
HILFEVORRICHTUNG
DISPOSITIF D'ASSISTANCE

(30) Priority: 31.08.2007 JP 2007226981
(43) Date of publication of application: 12.05.2010
(73) Proprietor: Terumo Kabushiki Kaisha, Tokyo 151-0072 (JP)
(72) Inventor: SEI, Naohisa, Tokyo 151-0072 (JP)
(74) Representative: TBK
(86) International application number: PCT/JP2008/065496
(87) International publication number: WO 2009/028645

(56) References cited:
- EP-A1- 0 132 940
- FR-A1- 2 699 083
- JP-A- 10 295 675
- JP-A- 2000 237 171
- JP-A- 2001 245 875
- JP-A- 2005 279 058
- JP-A- 2006 223 320
- JP-B2- 3 659 919
- US-A- 5 292 325

## Description

### TECHNICAL FIELD

The present invention relates to an assistance device for puncturing the skin with a puncture device and for measuring a desired blood component with a measurement device.

### BACKGROUND ART

Diabetic patients are recommended to perform a daily self-management of diabetes for keeping their blood glucose at an appropriate level. A diabetic patient manages his or her own blood glucose levels by puncturing the skin of a finger, the palm or the like with a puncturing tool in order to draw a small amount of blood from the skin, remove a blood sample with a blood sampler of a measurement tool, and then measure the blood glucose level of the blood sample with a blood glucose meter. The blood glucose level may be measured optically or electrically. After the blood glucose level has been measured, it is customary for the diabetic patient to inject a suitable amount of insulin with an insulin-filled syringe, in accordance with a doctor's instructions.

Many diabetics are elderly people, and some of them have disabled hands and fingers. Some diabetic patients have disabled hands and fingers because of certain complicating disorders, or may also suffer partial or complete loss of eyesight. Such handicapped diabetics may find it difficult to handle the puncture device and the measurement tool.

In view of the above difficulties, in Patent Document 1, the present applicant has proposed an assistance device including a measurement tool case holder for taking out a measurement tool, a puncture needle holder in the form of a hole for holding, in an upstanding manner, a puncture needle to be used while mounted in a puncture device, a cap separator for removing a cap from the puncture needle, and a puncture needle separator for removing the puncture needle from a puncture device. These functional components are arranged in an order in which they are used. The assistance device makes it easy for a diabetic patient to handle components which are used during management of the patient's blood glucose levels.

There have been demands from patients who have disabled hands and fingers, or who can use only one hand, for improved handling of the blood glucose meter and the puncture device, not only when the measurement tool and the puncture needle are installed, but also when the blood glucose meter and the puncture device are put to use after the measurement tool and the puncture needle have been installed thereon.

Specifically, the puncture device is required to reliably puncture a desired skin region at a desired blood sampling region, such as a fingertip, the palm, or the like. The blood glucose meter is required to be used in a so-called spot application practice, where the blood glucose meter is held in one hand and blood from a punctured fingertip of the other hand is applied to a blood sampler of the blood glucose meter. However, such puncture and spot application practices are not easy to perform for patients who have disabled hands and fingers, or who can use only one hand. Also, it is difficult for patients who have trembling hands to hold the tool, and use of the tool tends to make them feel stressed.
Patent Document 1: Japanese Laid-Open Patent Publication No. 2001-245873

### DISCLOSURE OF THE INVENTION

It is an object of the present invention to provide an assistance device for increasing ease with which a puncture device can be handled during puncturing of a patient's skin, and also to facilitate handling of a measurement tool, such as a blood glucose meter, for measuring a desired blood component during a spot application practice.

According to the present invention, an assistance device for puncturing the skin with a puncture device having a puncture needle, and measuring a desired blood component with a measurement device on which a measurement tool having a blood sampler is mounted, comprises a main body and a lid openably and closably joined to the main body, wherein one of the main body and the lid has a puncture guide for positioning the puncture needle in alignment with a puncture position on the skin, and the other of the main body and the lid has a blood sampling guide for positioning the measurement tool in alignment with a region of the skin which is punctured by the puncture needle.

With the above arrangement, after the puncture needle has punctured the skin while the puncture guide on one of the main body and the lid positions the puncture needle, the measurement tool is spot-applied to blood that comes out from the punctured skin while the measurement tool is reliably positioned by the blood sampling guide. Accordingly, the assistance device allows even weak-sighted users or users with trembling hands, for example, to perform a puncture operation and to carry out a measurement operation reliably and easily.

The main body may include a bleeding accelerator for pressing the skin upon contact therewith in order to cause blood to flow out from the skin, the bleeding accelerator being of a tapered shape that is progressively greater in diameter in a direction toward the skin. The bleeding accelerator can cause a sufficient amount of blood to flow out for measurement in the event that the amount of blood that flows out only due to puncturing is not enough.

The assistance device may include a fixing means for fixing the main body to a blood sampling region. While the assistance device is reliably secured at a desired blood sampling region by the fixing means, the skin can be punctured and the measurement tool can be spot-applied to the blood.

The assistance device may further include a biasing means for biasing the lid in an opening direction, and a holding means for holding the lid in a closed state. The biasing means and the holding means allow the lid to be opened and closed easily.

When the lid is closed, a centerline of a hole of the puncture guide and a centerline of a hole of the blood sampling guide may be held in alignment with each other. Therefore, the puncture needle and the measurement tool may reliably be held and positioned in alignment with each other.

The cross section of a hole of one of the puncture guide and the blood sampling guide, which is provided on the lid, may be included within the cross section of a hole of the other of the puncture guide and the blood sampling guide, which is provided on the main body, in planar projection. Accordingly, the lid may be reliably opened and closed with respect to the main body.

The fixing means may comprise two curved arcuate ring members, which form an annular shape when the ring members engage with each other. One of the two ring members may have sawtooth engaging recesses for adjusting the diameter of the fixing means.

The main body may be mounted detachably on the fixing means, and the assistance device may further include a positioning means for positioning the main body with respect to the fixing means in a plurality of orientations.

### BRIEF DESCRIPTION OF THE DRAWINGS

FIG. 1 is a perspective view of an assistance device according to a first embodiment of the present invention;
FIG. 2 is a perspective view of the assistance device shown in FIG. 1 with a lid thereof being open;
FIG. 3 is an exploded perspective view of the assistance device shown in FIG. 1;
FIG. 4 is a perspective view of a blood glucose meter and a puncture device unit;
FIG. 5 is a sectional side elevational view showing the manner in which a measurement tool is inserted into a blood glucose meter shown in FIG. 4;
FIG. 6 is a side elevational view of a puncture device and a measurement tool unit;
FIG. 7 is a sectional side elevational view of the assistance device shown in FIG. 1;
FIG. 8 is an exploded sectional side elevational view of the assistance device shown in FIG. 1;
FIG. 9A is a bottom view of a main body of the assistance device shown in FIG. 1;
FIG. 9B is a plan view of a fixing ring of the assistance device shown in FIG. 1;
FIG. 10A is a fragmentary sectional side elevational view of the assistance device shown in FIG. 1 when the assistance device is affixed to a fingertip;
FIG. 10B is a fragmentary sectional side elevational view showing the manner in which, from the state shown in FIG. 10A, a puncture needle is held in abutment with the skin;
FIG. 11 is a sectional side elevational view showing the manner in which, from the state shown in FIG. 10B, the lid is opened and the main body is pressed by a finger;
FIG. 12A is a fragmentary sectional side elevational view showing the manner in which, from the state shown in FIG. 11, the measurement tool is inserted into a blood sampling guide of the assistance device;
FIG. 12B is a fragmentary sectional side elevational view showing the manner in which the measurement tool is moved from the state shown in FIG. 12A, and a tip end thereof is spot-applied to a blood droplet;
FIG. 13A is a side elevational view of a modification of the assistance device shown in FIG. 1;
FIG. 13B is a side elevational view of a further modification of the assistance device shown in FIG. 13A;
FIG. 14 is an exploded perspective view of another modification of the assistance device shown in FIG. 1;
FIG. 15 is a perspective view of an assistance device according to a second embodiment of the present invention;
FIG. 16 is a perspective view of the assistance device shown in FIG. 15, with a lid thereof being open;
FIG. 17 is a sectional side elevational view of the assistance device shown in FIG. 15;
FIG. 18 is a perspective view of another example of a blood glucose meter and a measurement tool;
FIG. 19 is a perspective view of another example of a puncture device and a puncture needle;
FIG. 20 is a perspective view of a modification of the assistance device shown in FIG. 15; and
FIG. 21 is a side elevational view of a modification of the fixing ring.

### BEST MODE FOR CARRYING OUT THE INVENTION

Preferred embodiments of assistance devices according to the present invention will be described in detail below with reference to the accompanying drawings.

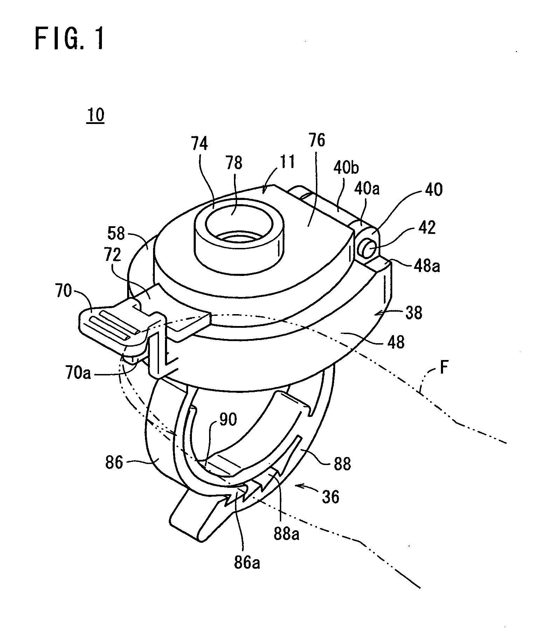

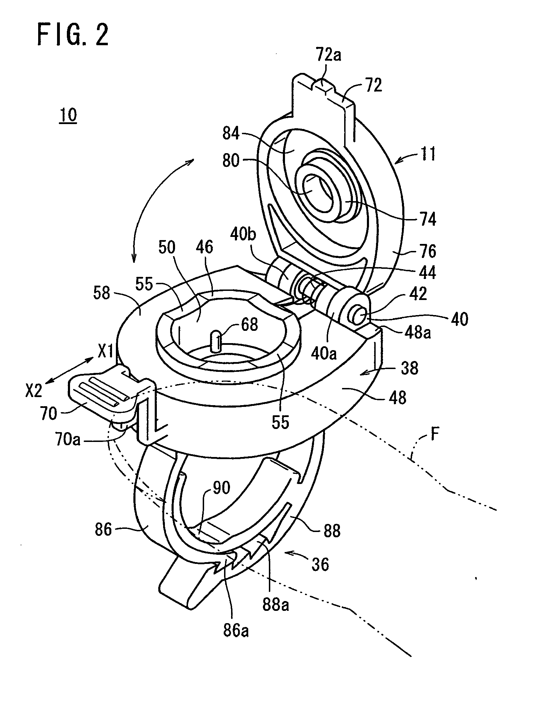

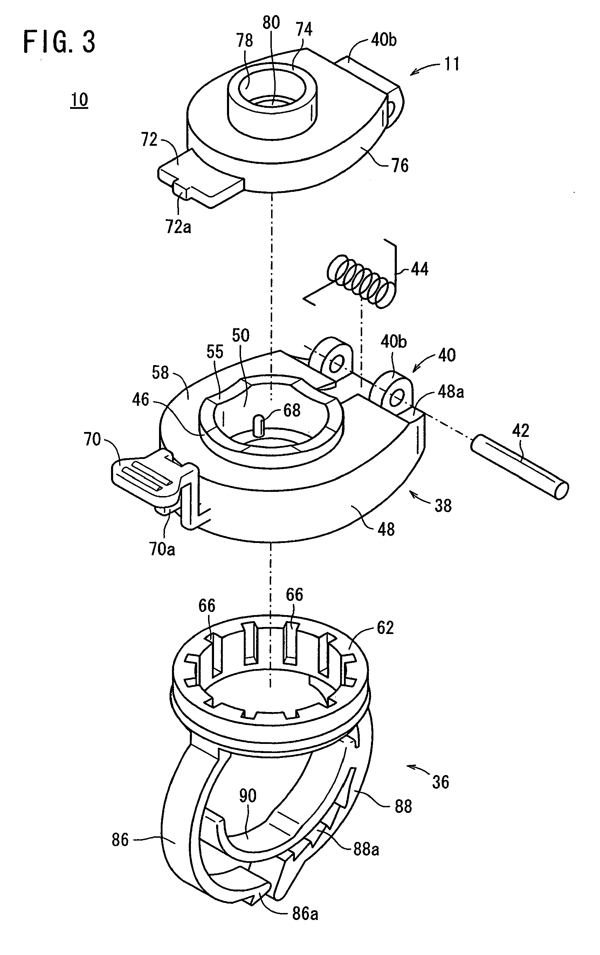

FIG. 1 is a perspective view of an assistance device 10 according to a first embodiment of the present invention, showing an example in which the assistance device 10 is used with a lid 11 thereof being closed. FIG. 2 is a perspective view of the assistance device 10 shown in FIG. 1 with the lid 11 thereof being open, and FIG. 3 is an exploded perspective view of the assistance device 10. The assistance device 10 comprises a device for guiding working units of a blood glucose meter 12 and a puncture device 14 toward a desired skin region, to thereby facilitate handling of the blood glucose meter 12 and the puncture device 14 when the blood glucose meter 12 is used as a measurement device for measuring a desired blood component, i.e., a blood glucose level, and also when the puncture device 14 is used to puncture the fingertip of a patient before the blood glucose level is measured by the blood glucose meter 12.

Prior to describing the assistance device 10 according to the present embodiment, the blood glucose meter 12 and the puncture device 14 will first be described below. The blood glucose meter 12 and the puncture device 14 are examples of instruments to which the assistance device 10 according to the present embodiment can be applied. The assistance device 10 can also appropriately be used with other measurement devices and puncture devices, according to other arrangements.

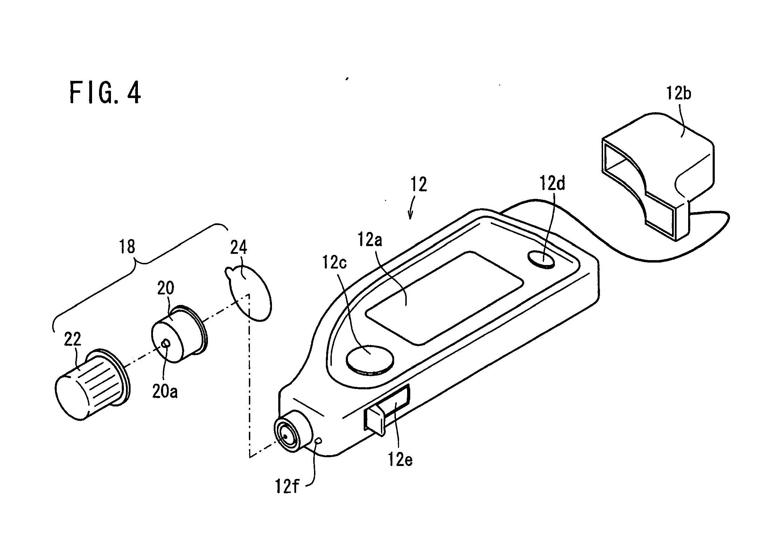

As shown in FIG. 4, the blood glucose meter 12 has a measurement tool 20 of a measurement tool unit 18 mounted on a tip end thereof, and serves to measure a blood glucose level and display the measured blood glucose level on a monitor 12a. The measurement tool unit 18 has the measurement tool 20 which acts as a test piece, a cylindrical measurement tool case 22 for housing the measurement tool 20 therein, and a seal 24 that hermetically seals the opening of the measurement tool case 22.

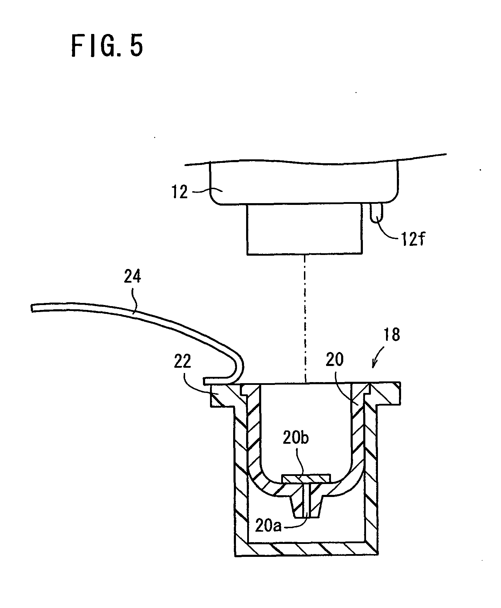

As shown in FIG. 5, the measurement tool 20 includes a capillary tube (blood sampler, blood spot applier) 20a, which is open at a tip end thereof, and a test paper 20b, which is disposed so as to close another opening of the capillary tube 20a. The test paper 20b holds a reagent that reacts with blood glucose.

As shown in FIGS. 4 and 5, when blood glucose is to be measured, the seal 24 is peeled off, and thereafter the measurement tool 20 is mounted on the blood glucose meter 12, from which a cap 12b has been removed. Then, blood is sampled through the capillary tube 20a, which serves as a blood sampler. The cap 12b is connected by a strap to the main body of the blood glucose meter 12.

The blood glucose meter 12 is turned on when a power supply switch 12c is pressed. The blood glucose meter 12 then automatically starts to measure blood when the blood is spot-applied thereto. The blood glucose meter 12 detects a change in the coloration of the blood, which is sampled on the test paper 20b of the measurement tool 20, and then performs predetermined calculations and displays the obtained blood glucose level on the monitor 12a. The measured result is recorded in an internal memory. A record of measured results can be displayed when a call button 12d is operated. After use, when a pin 12f is pushed out by moving a lever 12e, the measurement tool 20 is removed from the blood glucose meter 12, and the measurement tool 20 then is discarded according to a certain procedure.

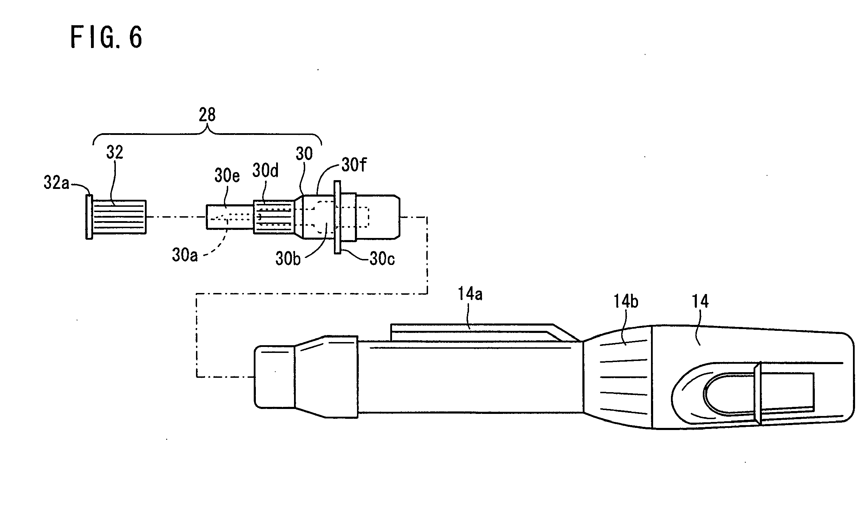

As shown in FIG. 6, the puncture device 14 is substantially in the shape of a rod. A puncture needle 30 of a puncture needle unit 28 is mounted on a tip end of the puncture device 14, whereupon the puncture needle 30 is used to puncture the skin and to draw a small amount of blood therefrom. The puncture needle unit 28 includes the puncture needle 30 and a cap 32 that covers the tip end of the puncture needle 30. The puncture needle 30 has a needle 30a and a hub 30b, which are disposed in a puncture needle case 30f, such that the hub 30b holds the needle 30a. The needle 30a is kept in a sterilized state by the cap 32, which is disposed at a front end, whereas the hub 30b is disposed at a rear end. The puncture needle case 30f is of a multi-stepped tubular shape and has a flange 30c. The puncture needle case 30f includes a second tip end portion 30e at the tip end of the puncture needle 30. The second tip end portion 30e is smaller in diameter than the measurement tool 20.

When the puncture needle 30 is mounted in the puncture device 14, the puncture needle 30 is held therein with an internal spring in the puncture device 14 being in a compressed state. When a puncture button 14a is pressed, the spring is released to cause the needle 30a to project instantaneously and puncture the skin. The distance that the needle 30a projects is adjustable by operating a dial 14b. After use, the puncture needle 30 is removed from the puncture device 14, and the puncture needle 30 is discarded according to a certain procedure. The cap 32 is of a tubular shape and includes a flange 32a on a tip end thereof.

The assistance device 10 according to the present embodiment will be described below.

As shown in FIGS. 1 through 3, the assistance device 10 comprises a fixing ring 36, which serves as a fixing means for fixing the assistance device 10 at a desired blood sampling region (e.g., a fingertip), a main body 38 joined to an upper portion of the fixing ring 36, and a lid 11 openably and closably (swingably) joined to the main body 38 by a hinge assembly 40. All of the components of the assistance device 10, except for a shaft pin 42 and a coil spring 44 of the hinge assembly 40, are made of a resin material, such as polypropylene, for example. All of the components of the assistance device 10, including the hinge assembly 40, may also be made of a resin material. The hinge assembly 40 may be constructed as a thin resin hinge joined to the main body 38 and the lid 11.

The main body 38 comprises a substantially central tubular blood sampling guide 46 and a hollow substantially cylindrical outer edge 48 disposed around the blood sampling guide 46. The main body 38 performs a function to position and guide the measurement tool 20 mounted on the blood glucose meter 12 to a blood drawing region of the skin, which has been punctured by the puncture device 14.

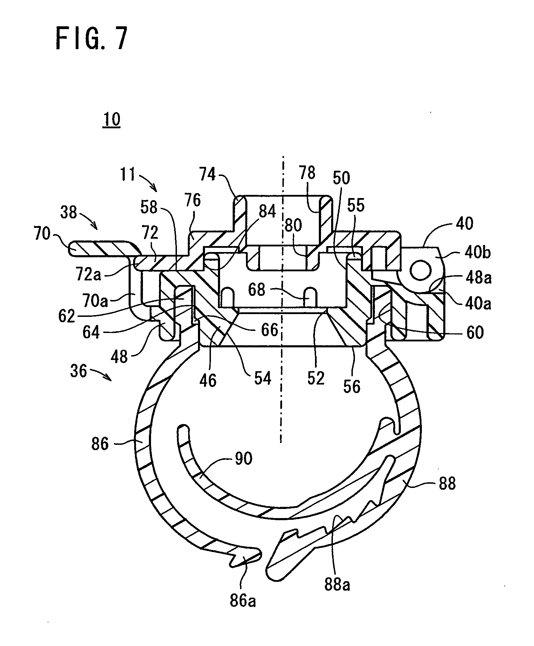

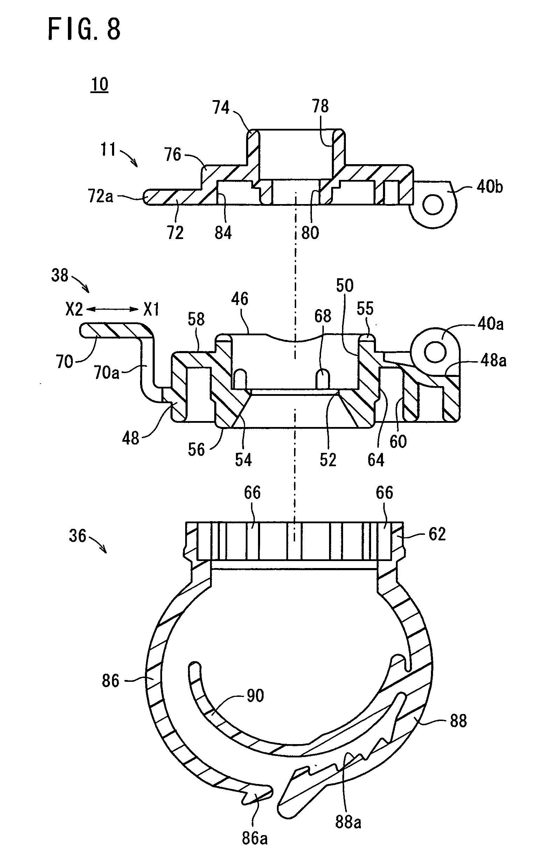

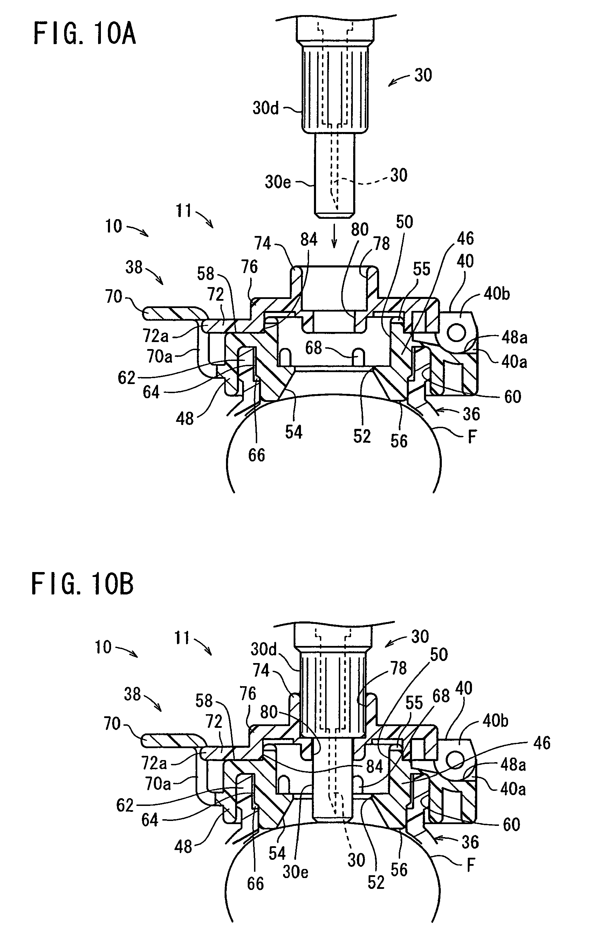

As shown in FIGS. 7 and 8, the blood sampling guide 46 has a multi-stepped hole defined therein, which includes in succession axially from the lid 11, a guide hole 50 for receiving the measurement tool 20 inserted therein, a hole 52 smaller in diameter and shorter than the guide hole 50, and a tapered hole 54, which is progressively greater in diameter downwardly from the hole 52. The tapered hole 54 has a lower end opening (larger diameter side) defined by an annular abutment surface 56, which serves as a skin abutment surface and abuts against the skin of a person whose blood is to be measured when the assistance device 1 is used (see FIG. 10A).

An annular groove 60 is defined around the hole 52 and the tapered hole 54 of the blood sampling guide 46, as well as behind an upper surface 58 of an outer edge 48. When the fixing ring 36 is joined to the main body 38, the fixing ring 36 has a hollow cylindrical joint 62 fitted into the annular groove 60.

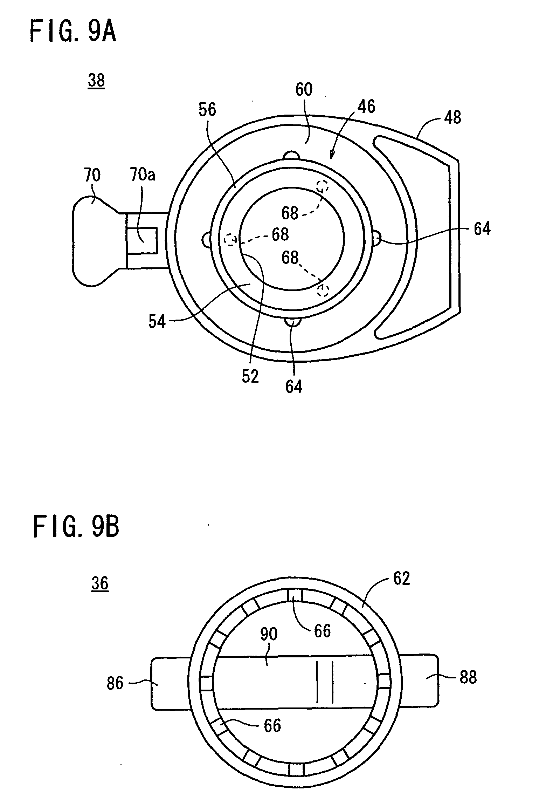

As shown in FIGS. 9A and 9B, the blood sampling guide 46, which is positioned within the annular groove 60 of the main body 38, has a plurality of (in the present embodiment, four equally spaced) positioning ribs (positioning means) 64 on an outer circumferential surface thereof. The joint 62 of the fixing ring 36 has a plurality of (in the present embodiment, twelve equally spaced) positioning grooves (positioning means) 66, which are combinable with the positioning ribs 64. When the positioning ribs 64 engage within the positioning grooves 66, the fixing ring 36 is joined at a desired angle to the main body 38. Stated otherwise, the direction of the main body 38 with respect to the fixing ring 36 can be changed as desired.

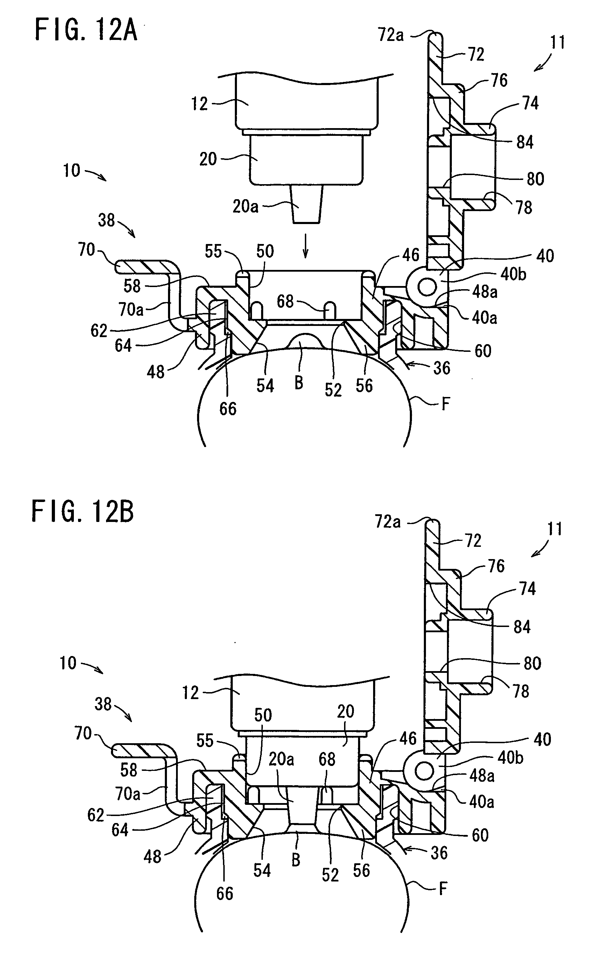

As shown in FIG. 12B, the inside diameter of the guide hole 50 is set slightly greater than the outside diameter of the measurement tool 20 so as to allow the measurement tool 20 to be positioned reliably. Three equally spaced, upwardly projecting protrusions 68 are disposed on the bottom of the guide hole 50 (the upper surface of a flange that defines the hole 52) in order to adjust the capillary tube 20a on the tip end of the measurement tool 20 to a suitable height out of contact with the skin, so that blood can appropriately be spot-applied to the capillary tube 20a (see FIG. 12B).

As shown in FIG. 2, the blood sampling guide 46, which projects from the upper surface 58 of the outer edge 48, has a predetermined number of (in the present embodiment, four) curved indicator slots 55 defined on an annular upper surface. The indicator slots 55 can manually be felt by a weak-sighted patient in order to confirm the position and orientation of the guide hole 50 of the main body 38.

The outer edge 48 that surrounds the blood sampling guide 46 has an end 48a, which is recessed from the upper surface 58 and which has a main body hinge 40a that forms part of the hinge assembly 40 through which the shaft pin 42 is inserted. The outer edge 48 also has a holding means 70 disposed opposite to the main body hinge 40a. The holding means 70 holds the lid 11 in a closed state. The holding means 70 comprises a lever integrally combined with the outer edge 48, and which is normally in a reference position. When the lid 11 is closed, the holding means 70 temporarily is swung in the direction indicated by the arrow X2 by a holder tongue 72 that projects from the lid 11, and thereafter, the holding means 70 returns to the reference position in the direction indicated by the arrow X1. The holding means 70 has a central slit 70a which holds a tooth 72a on the tip end of the holder tongue 72, thereby holding the lid 11 in the closed position. For releasing the lid 11, the holding means 70 is pressed lightly downward and is tilted in the direction indicated by the arrow X2, thus allowing the tooth 72a to be released from the slit 70a. The lid 11 is automatically opened under the bias of the coil spring (biasing means) 44 of the hinge assembly 40.

The lid 11 is openably and closably coupled to the main body 38 by a lid hinge 40b, which forms another part associated with the main body hinge 40a and through which the shaft pin 42 is inserted. The lid 11 comprises a substantially central tubular puncture guide 74 and a hollow substantially cylindrical outer edge 76 disposed around the puncture guide 74. The lid 11 performs a function to position and guide the puncture needle 30 that is mounted in the puncture device 14, when the puncture device 14 punctures the skin prior to measurement of blood by the blood glucose meter 12.

As shown in FIGS. 7 and 8, the puncture guide 74 has a two-stepped hole defined therein, which includes in succession axially from above, a first guide hole 78, and a second guide hole 80 for receiving the puncture needle 30 inserted therein. The first guide hole 78 and the second guide hole 80 have respective inside diameters for guiding a first tip end portion 30d of the puncture needle 30 and a small-diameter second tip end portion 30e (see FIG. 6) thereof, which is positioned more closely to the tip end than the first tip end portion 30d. By appropriately guiding the puncture needle case 30f, the puncture guide 74 guides the needle 30a to a puncture position.

An annular groove 84 is defined around the second guide hole 80 of the puncture guide 74 and behind an upper surface 82 of an outer edge 76. When the lid 11 is closed in a position over the main body 38, the annular groove 84 functions as a clearance, in which the projecting end of the blood sampling guide 46 of the main body 38 can be accommodated.

As shown in FIG. 7, when the lid 11 is closed toward the main body 38, the projecting end of the blood sampling guide 46 of the main body 38, with the indicator slots 55 defined therein, is inserted into the annular groove 84, and the lower surface of the lid 11 is seated on the upper surface 58 of the outer edge 48 of the main body 38. While the holding means 70 holds the holder tongue 72, the lid 11 is closed over the main body 38 and is held in intimate contact therewith. At this time, the first guide hole 78 and the second guide hole 80 of the lid 11, the guide hole 50, the hole 52, and the tapered hole 54 of the main body 38 are arranged in coaxial alignment with each other.

When the fixing ring 36 is fitted over a fingertip of the user (patient or the like), the fixing ring 36 serves as a fixing means for fixing the assistance device 10 onto the fingertip. The fixing ring 36 comprises a pair of curved arcuate ring members 86, 88 extending downwardly from the joint 62 that is joined to the main body 38. The fixing ring 36 forms an annular shape when the ring members 86, 88 engage with each other. More specifically, when an engaging tooth 86a on a tip end portion of the ring member 86 engages within any one of sawtooth engaging recesses 88a on the other ring member 88, the fixing ring 36 can be wound around and fixed to the fingertip at a desired diameter. The ring member 88 includes a protective arm 90 disposed inwardly from the engaging recesses 88a for preventing the sawtooth engaging recesses 88a from coming into contact with the fingertip of the user and thus causing the user to feel pain.

The assistance device 10 is basically constituted as described above. An example in which the assistance device 10 is used will be described below.

First, the user (patient) presses an unused measurement tool unit 18, from which the seal 24 has been removed, against the tip end of the blood glucose meter 12 and installs the measurement tool 20 on the blood glucose meter 12. The user then removes the measurement tool case 22. At this time, the pin 12f is accommodated in the blood glucose meter 12. Then, an unused puncture needle unit 28 is mounted on the tip end of the puncture device 14. The hub 30b of the puncture needle 30 compresses the spring (not shown) in the puncture device 14 and becomes engaged with a predetermined engaging portion therein, thereby making the puncture needle 30 ready for puncture. The user then removes the cap 32 from the puncture needle 30.

Then, the user fixes the assistance device 10 to a desired blood sampling region, e.g., the fingertip of an index finger or a middle finger. More specifically, as shown in FIGS. 1 and 10A, the fingertip F is inserted within the ring members 86, 88 of the fixing ring 36 of the assistance device 10, such that the ball of the fingertip F faces toward the main body 38. Then, the engaging tooth 86a is brought into engagement with one of the engaging recesses 88a in order to adjust the ring members 86, 88, thereby securing the assistance device 10 to the fingertip F. Since the ring members 86, 88 can be tightened or loosened simply by pushing them from opposite sides, the user can easily and reliably secure the assistance device 10 to the fingertip F at a desired position, even if the user is weak-sighted.

After the assistance device 10 has been prepared for use, the puncture device 14 is used to puncture the fingertip F. According to the present embodiment, when or after the assistance device 10 has been secured to the fingertip F, the lid 11 is placed in a closed position on the main body 38.

The tip end of the puncture needle 30 mounted in the puncture device 14 is inserted through the holes in the assistance device 10 into abutment against the skin of the ball of the fingertip F. More specifically, as shown in FIG. 10B, the tip end of the puncture needle 30 is inserted from the first guide hole 78 of the puncture guide 74 of the lid 11, thereby causing the second tip end portion 30e to pass through the first guide hole 78 into the second guide hole 80. The tip end of the second tip end portion 30e is guided by the second guide hole 80, so as to be positioned in abutment with the skin of the fingertip F, which serves as a blood sampling region (see FIG. 10B). In other words, the puncture needle 30 is appropriately arranged in both position and height with respect to a predetermined position beneath the fingertip F.

In order for the second guide hole 80 of the puncture guide 74 to accurately guide and position the second tip end portion 30e of the puncture needle 30, the second guide hole 80 has an inside diameter of about 4.2 mm, provided that the second tip end portion 30e has an outside diameter of 4 mm. Since the puncture needle 30 is positioned essentially between the second guide hole 80 and the second tip end portion 30e, the clearance between the first guide hole 78 and the first tip end portion 30d may be slightly greater than the clearance (about 0.2 mm) between the second guide hole 80 and the second tip end portion 30e. For example, if the outside diameter of the first tip end portion 30d is 5.6 mm, then the inside diameter of the first guide hole 78 is about 6 mm. Thus, the puncture needle 30 can easily be inserted into the first guide hole 78 and the second guide hole 80, in order to easily and reliably bring the puncture position of the needle 30a that projects from the tip end of the puncture needle 30 into a substantially central position in the tapered hole 54 (see FIG. 10B).

When the puncture button 14a is pressed, while the puncture needle 30 is positioned in a predetermined position by the assistance device 10, the coil spring inside the puncture device is released from a compressed state, thereby forcing the hub 30b and the needle 30a to project forward instantaneously. The needle 30a punctures the fingertip F within the tapered hole 54 in the main body 38, whereby a small amount of blood flows out from the fingertip F. The hub 30b and the needle 30a are then retracted into the puncture needle 30 by a return spring, not shown.

After the puncture device 14 has been pulled out from the assistance device 10, the holding means 70 is operated to open the lid 11 from the main body 38. With the assistance device 10, the lid 11 can be opened simply under the bias of the coil spring 44, by turning the holding means 70 in the direction indicated by the arrow X2.

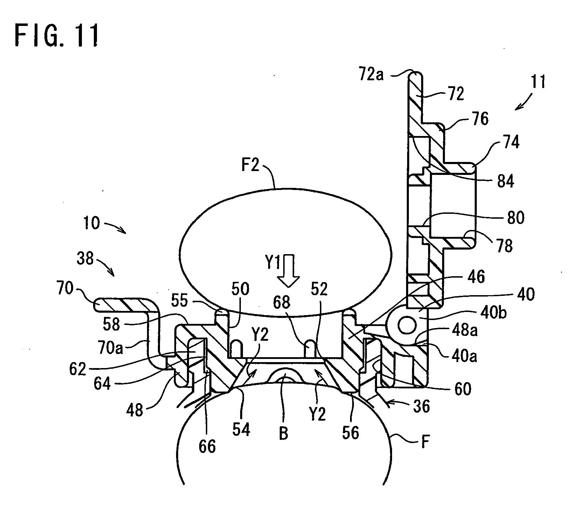

Then, as shown in FIG. 11, the main body 38 is pressed by a finger F2 of the other hand in the direction indicated by the arrow Y1, so as to compress the region punctured by the puncture needle 30 and the surrounding skin with the abutment surface 56 and the inner surface of the tapered hole 54. As a result, forces are applied to the punctured region of the skin along the tapered hole 54 in the direction indicated by the arrow Y2. Accordingly, a sufficient amount of blood is pressed out from the punctured region of the skin of the fingertip F, thereby forming a blood droplet B, which contains enough blood suitable for measurement by the blood glucose meter 12. The fingertip F may also be compressed by tightening the ring members 86, 88 of the fixing ring 36.

Therefore, the main body 38 of the assistance device 10, and in particular the tapered hole 54 and the abutment surface 56, can function as a bleeding accelerator for accelerating bleeding from the punctured region. Inasmuch as the hole inside of the abutment surface 56, which compresses the fingertip F, contains the tapered hole 54 that provides a sufficient space, the blood droplet B formed as described above is effectively prevented from contacting the assistance device 10 itself. If the blood were applied to the assistance device 10, then the blood would tend to permeate between the abutment surface 56 and the skin, and thus fail to produce a suitable blood droplet B.

Subsequently, the tip end of the measurement tool 20, which is mounted on the blood glucose meter 12, is applied to the blood droplet B formed by puncturing the skin. More specifically, as shown in FIGS. 12A and 12B, the tip end of the measurement tool 20 is inserted from the guide hole 50 of the blood sampling guide 46 of the main body 38, so that the capillary tube 20a, which serves as a blood sampler at the tip end thereof, is spot-applied to the blood droplet B formed in the tapered hole 54. At this time, the blood passes through the capillary tube 20a and impregnates the test paper 20b (see FIG. 5), thereby reacting with the reagent and producing a color, based on which the blood glucose level can be automatically measured optically. When the measured blood glucose level is displayed on the monitor 12a, the user can confirm his or her blood glucose level.

With the assistance device 10, the puncture guide 74 (the first guide hole 78 and the second guide hole 80) for guiding and positioning the puncture needle 30, and the blood sampling guide 46 (the guide hole 50 and the tapered hole 54) for guiding and positioning the measurement tool 20 are in coaxial alignment with each other, as described above. Therefore, the blood can be spot-applied to the capillary tube 20a while the capillary tube 20a of the measurement tool 20 is reliably positioned substantially at a central position on the blood droplet B, which is formed when the puncture needle 30 punctures the fingertip F. Consequently, blood glucose levels can reliably and easily be measured.

The protrusions 68 may be set to an appropriate height to allow the capillary tube 20a at the tip end of the measurement tool 20 to reliably contact only the blood, without being abutted against the skin (see FIG. 12B). The distance between the tip end of the blood sampling nozzle (i.e., the tip end of the measurement tool 20, or more specifically the tip end of the capillary tube 20a) and the skin should preferably be in a range from about 0.1 to 1.0 mm. When the assistance device 10 is manufactured, the protrusions 68 may be set with a slightly larger height, to enable adjustment of the height of the protrusions depending on the conditions under which the measurement tool 20 will be used and the fingertip F of the user. The height of the capillary tube 20a also can be set and changed to an appropriate value. After the puncture needle 30 has punctured the fingertip F, the measurement tool 20, which is held in abutment with the protrusions 68, may be compressed, rather than compressing the main body 38 with the finger F2 of the other hand, in order to cause blood to flow out from the punctured region, and for spot-applying the blood to the capillary tube 20a. For example, even if the main body 38 is not sufficiently compressed with the finger F2, or if the measurement unit 20 is not suitably spot-applied to the blood, the main body 38 may be compressed by the measurement tool 20 in order to cause the blood to flow out from the fingertip F for reliably measuring the blood glucose level.

Once the above measuring process is finished, one of the ring members 88 (86) of the fixing ring 36 of the assistance device 10 is pulled in order to loosen the ring members 86, 88 away from each other, and the assistance device 10 is taken off from the fingertip. The puncture needle 30 is removed from the puncture device 14, and the measurement tool 20 on the tip end of the blood glucose meter 12 is inserted into the measurement tool case 22. The lever 12e is operated to eject the pin 12f, thereby removing the measurement tool 20 from the blood glucose meter 12, and the measurement tool 20 is covered with the seal 24. Then, the puncture needle 30, the measurement tool 20, and the measurement tool case 22 are discarded according to a certain procedure.

As described above, the assistance device 10 according to the present embodiment can reliably be fixed to a desired blood sampling region from which blood is sampled. With the assistance device 10, the puncture guide 74 that guides and positions the puncture needle 30 and the blood sampling guide 46 that guides and positions the measurement tool 20 are in coaxial alignment with each other. Therefore, the puncture position of the puncture needle 30 and the measurement (spot application) position of the measurement tool 20 can accurately be brought into alignment with each other. The assistance device 10 thus allows even weak-sighted users or users with trembling hands, for example, to perform puncture and measurement operations reliably and easily.

When the lid 11 is closed, since a centerline of the holes (the first guide hole 78 and the second guide hole 80) of the puncture guide 74 in which the puncture needle 30 is inserted and a centerline of the holes (the guide hole 50, the hole 52, and the tapered hole 54) of the blood sampling guide 46 in which the measurement tool 20 is inserted are aligned with each other, the puncture needle 30 and the measurement tool 20 can reliably be held in positional alignment with each other. Furthermore, when viewed in planar projection, inasmuch as the cross section of the holes of the puncture guide 74, which is provided in the lid 11 and in which the puncture needle 30 is inserted, is included within the cross section of the holes of the blood sampling guide 46, which are provided in the main body 38 and in which the measurement tool 20 is inserted, the lid 11 can reliably and easily be opened and closed with respect to the main body 38.

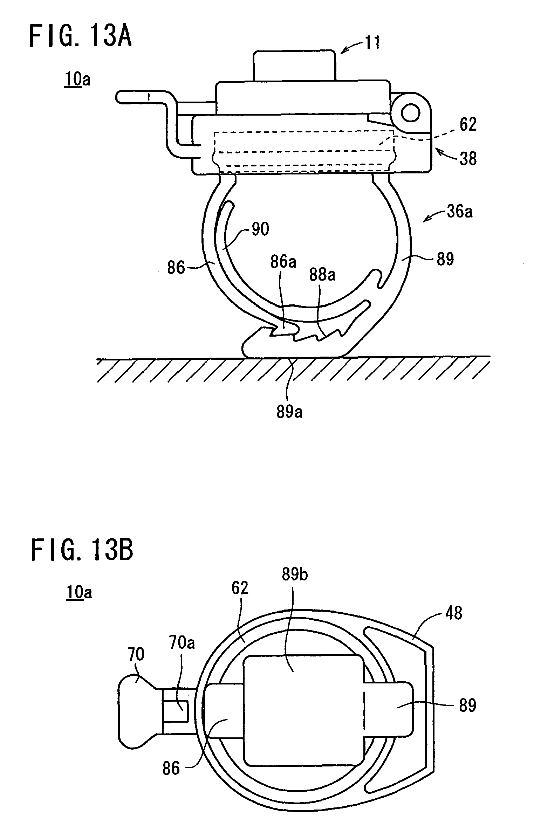

As shown in FIG. 13A, the assistance device 10 according to the present embodiment may be modified into an assistance device 10a in which the fixing ring 36 is changed to a fixing ring 36a. The fixing ring 36a has a ring member 89 including a placement surface 89a, which is formed by a substantially flat surface on a portion of the outer circumferential surface thereof. When the assistance device 10a is used, the placement surface 89a may be abutted against the upper surface of a table or the like in order to keep the assistance device 10a in a stable attitude. Therefore, the puncture device 14 and the blood glucose meter 12 can be handled with greater ease. As shown in FIG. 13B, the placement surface 89a may be modified to provide a placement surface 89b which is wider than the ring member 89, for thereby making the assistance device 10a even more stable. The above-described assistance device 10 may also have the ring member 88, the outer circumferential surface of which is made partially wider, similar to the case of the placement surface 89b, so that the assistance device 10 can be positioned with greater stability.

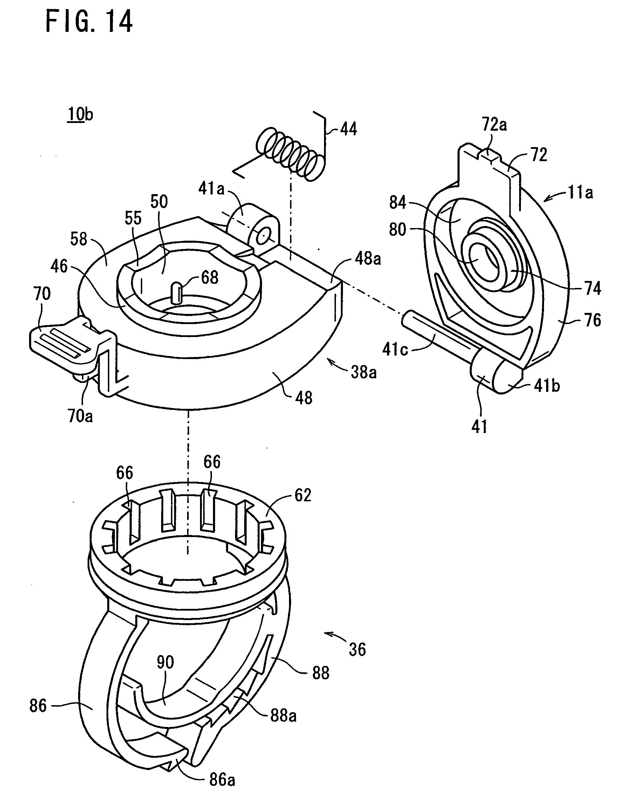

The lid 11 may be joined to the main body 38 by a structure other than the structure using the hinge assembly 40, which was described above. For example, as shown in FIG. 14, an assistance device 10b has a lid 11a and a main body 38a, which are detachable and openably and closably assembled together by means of a hinge assembly 41. The main body 38a has a main body hinge 41a. The lid 11a has a lid hinge 41b and a shaft pin 41c formed integrally therewith, which is inserted into the main body hinge 41a so that the main body 38a and the lid 11a are detachably assembled together. Since the main body 38a and the lid 11a are replaceable individually, the puncture guide and the blood sampling guide can easily be replaced by those having different shapes, depending on the type of puncture needle and measurement tool that are used. Naturally, when either one of the main body 38a or the lid 11a becomes damaged, it can easily be replaced.

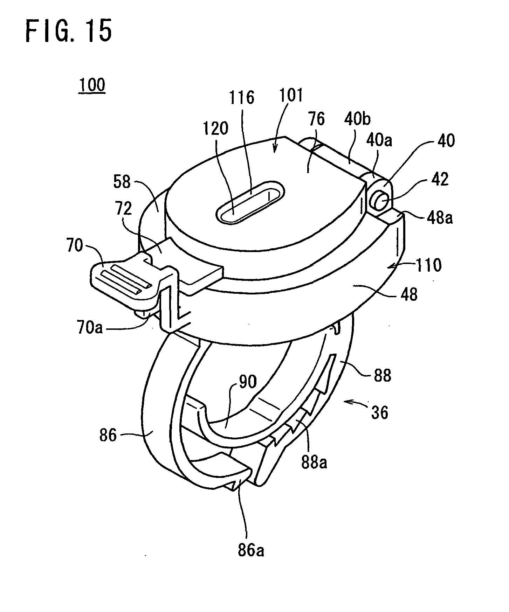

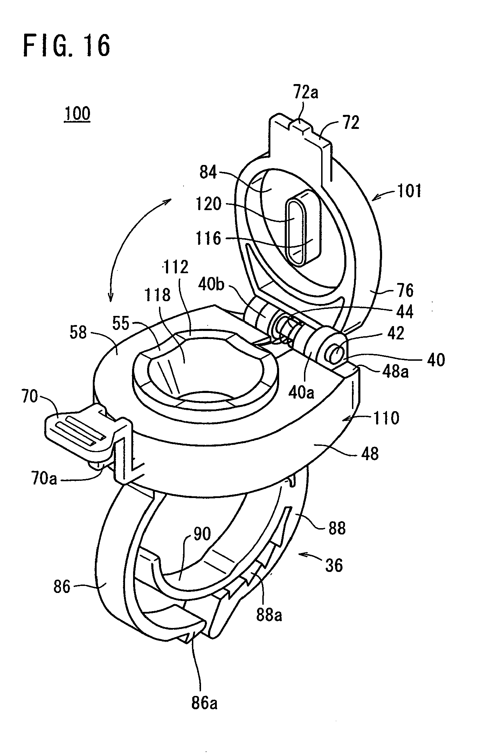

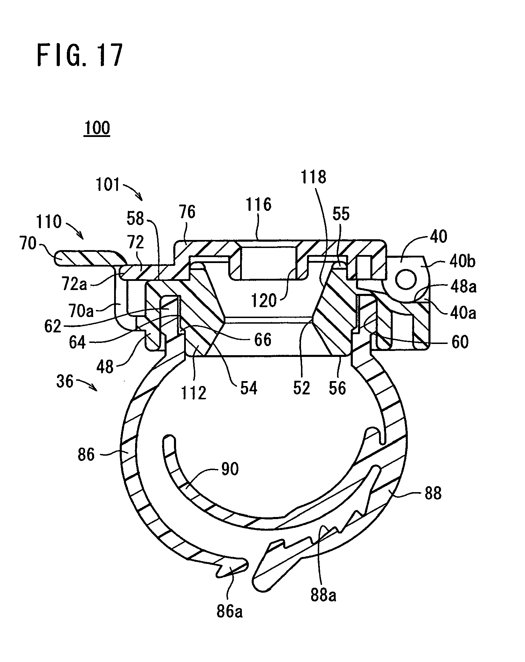

An assistance device 100 according to a second embodiment will be described below. FIG. 15 is a perspective view of the assistance device 100 according to the second embodiment, showing an example in which the assistance device 100 is used with a lid 101 thereof being closed. FIG. 16 is a perspective view of the assistance device 100 shown in FIG. 15 with the lid 101 thereof being open. FIG. 17 is a sectional side elevational view of the assistance device 100 shown in FIG. 15. Reference characters shown in FIG. 15, etc., which are identical to those shown in FIGS. 1 through 14 denote identical or similar components, and such features will not be described in detail below, since they provide identical or similar functions and effects.

The assistance device 100 is used effectively with a blood glucose meter 102 and a puncture device 104, which are slightly different in structure than the aforementioned blood glucose meter 12 and puncture device 14 (see FIGS. 4 and 6).

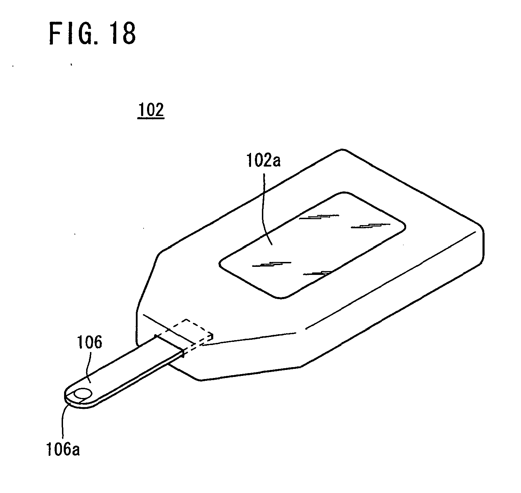

As shown in FIG. 18, instead of the measurement tool 20 of the blood glucose meter 12 (see FIG. 4), the blood glucose meter 102 includes a measurement tool 106 mounted on its tip end, which comprises an electrode sensor in the form of an elongate strip. The blood glucose meter 102 measures blood glucose levels by spot-applying the measurement tool 106 to blood, and then displays the measured blood glucose level on a monitor 102a.

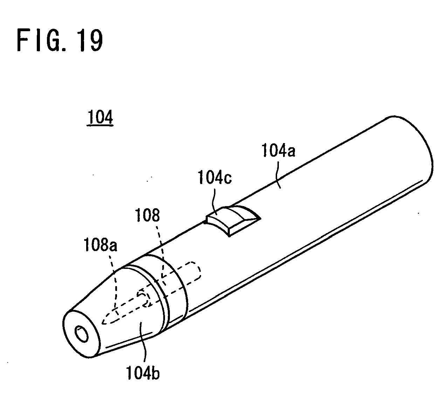

As shown in FIG. 19, the puncture device 104 is substantially in the shape of a rod, similar to the case of the puncture device 14 (see FIG. 6). When a cap 104b coupled to the tip end of a barrel 104a is removed, a puncture needle 108 is mounted in the tip end of the barrel 104a, which then is covered again with the cap 104b. Then, a puncture button 104c is pressed to force a needle 108a, which is disposed in the tip end of the barrel 104a, to project from a hole in the tip end of the cap 104b and thereby puncture the skin. The cap 104b has a cross-sectional area that is greater than the cross-sectional area of the measurement tool 106.

In order for the assistance device 100 to match the configuration of the measurement tool 106 of the blood glucose meter 102, as well as the configuration of the puncture needle 108 of the puncture device 104, the assistance device 100 includes a puncture guide 112 on a main body 110 thereof, which replaces the main body 38 of the assistance device 10, and a blood sampling guide 116 on a lid 101, which replaces the lid 11.

The main body 110 essentially has a structure in which the blood sampling guide 46 of the main body 38 is converted into the puncture guide 112.

As shown in FIG. 17, the puncture guide 112 has a multi-stepped hole defined therein which includes, in succession axially from the lid 101, a tapered guide hole 118, which becomes progressively smaller in diameter downwardly along the direction in which the puncture device 104 is inserted, a hole 52, and another tapered hole 54. The tapered guide hole 118 is complementary in shape to the tapered shape of the tip end of the cap 104b of the puncture device 104, and functions to guide the puncture needle 108 mounted in the puncture device 104 to a substantially central position in the tapered hole 54.

The lid 101 essentially has a structure in which the puncture guide 74 of the lid 11 is converted into the blood sampling guide 116. The blood sampling guide 116 has a slit-like guide hole 120 defined therein, which has a substantially rectangular cross-sectional shape for guiding and positioning the measurement tool 106, which is in the form of an elongate strip mounted on the tip end of the blood glucose meter 102. The tapered guide hole 118, the hole 52, and the tapered hole 54 of the puncture guide 112 in the main body 110, as well as the guide hole 120 of the blood sampling guide 116 of the lid 101, have respective centerlines that are held in coaxial alignment with each other. In other words, a central point 106a on the tip end of the measurement tool 106 and a centerline of the cap 104b are guided in a coaxial manner.

When the assistance device 100 according to the present embodiment is used, at first the lid 101 is opened. Then, the puncture device 104 with the puncture needle 108 thereon is guided into a desired position by the puncture guide 112 of the main body 38, thereby bringing the tip end face of the cap 104b into abutment against the skin. After the skin has been punctured, the lid 101 is closed, and the measurement tool 106 is guided accurately by the blood sampling guide 116 of the lid 101 to a position on the skin that has been punctured by the puncture needle 108 and from which blood emerges. As with the assistance device 10 according to the first embodiment described above, the assistance device 100 allows even weak-sighted users, or users with trembling hands, for example, to perform puncture and measurement operations reliably and easily.

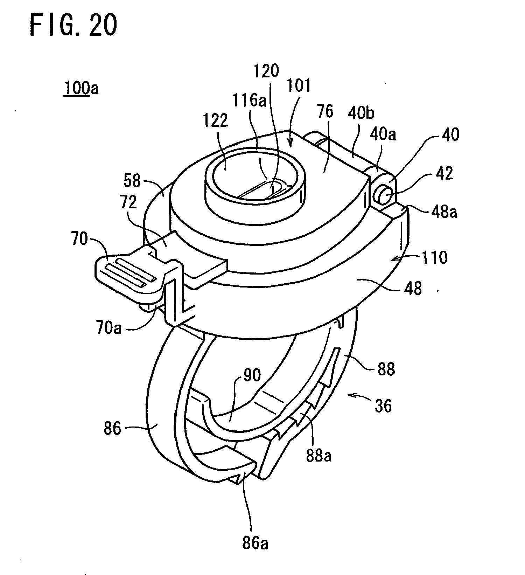

As shown in FIG. 20, the assistance device 100 according to the present embodiment may be modified to provide an assistance device 100a, in which the blood sampling guide 116 of the lid 101 is converted into a blood sampling guide 116a having an insertion guide 122 in the form of a tapered recess for insertion of the measurement tool 106. The assistance device 100a allows the measurement tool 106, which is mounted on the blood glucose meter 102, to be inserted more easily into the guide hole 120 through the insertion guide 122. Therefore, the assistance device 100a allows the blood glucose meter 102 to be handled with greater ease. The other guides of the assistance devices 10, 100, i.e., the blood sampling guide 46 and the puncture guides 74, 112, may also be provided with insertion guides, similar to the insertion guide 122.

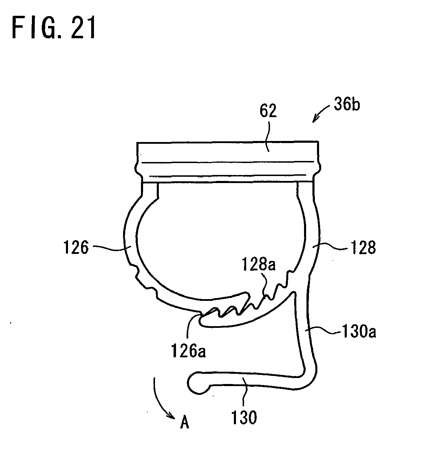

The fixing ring 36 for fixing the assistance devices 10, 100 to the blood sampling region may be replaced with a fixing ring 36b having ring members 126, 128 and a fixation canceling lever 130, as shown in FIG. 21. When a side portion 130b of the fixation canceling lever 130 is pressed, the ring members 126, 128 are brought into engagement with each other, thereby easily securing the assistance device in position. The ring member 126 has engaging teeth 126a on a tip end portion thereof, and the other ring member 128 has engaging recesses 128a for engagement with the engaging teeth 126a. When the fixation canceling lever 130 is pulled in a direction indicated by the arrow A, fixation of the assistance device can easily be canceled. The fixing ring, which serves as the fixing means, may have a greater diameter, thus making it possible for the assistance device to be secured to an arm of the user, etc. Instead of the fixing rings 36, 36a, 36b, the fixing means may comprise a simple band or the like.

The measurement device (blood glucose meter), the puncture device, the measurement tool, and the puncture needle may be of any of various shapes apart from those described above. In such a case, the puncture guide and the blood sampling guide may be changed in shape as necessary. The puncture needle 30 is not limited to one having a needle and a case, and the case may be dispensed with. The puncture device and the puncture needle may be combined integrally with each other.

Rather than the blood glucose meter, the measurement device, which is used with the assistance devices 10, 100, may be a measurement device for measuring inorganic ions, hemoglobin, etc., such as protein, cholesterol, alcohol, sodium, etc., as a component in the blood to be measured.

The assistance device according to the present invention is not limited to the above-described embodiments, but may employ various alternative and/or additional arrangements without departing from the scope of the present invention.

## Claims

1. An assistance device for puncturing skin with a puncture device (14, 104) having a puncture needle (30, 108), and measuring a desired blood component with a measurement device on which a measurement tool (20, 106) having a blood sampler is mounted, comprising:
a main body (38, 38a, 110); and
a lid (11, 11a, 101) openably and closably joined to said main body (38, 38a, 110),
wherein one of said main body (38, 38a, 110) and said lid (11, 11a, 101) has a puncture guide (74, 112) for positioning said puncture needle (30, 108) in alignment with a puncture position on the skin, and the other of said main body (38, 38a, 110) and said lid (11, 11a, 101) has a blood sampling guide (46, 116, 116a) for positioning said measurement tool (20, 106) in alignment with a region of the skin which is punctured by said puncture needle (30, 108).

2. An assistance device according to claim 1, wherein said main body (38, 38a, 110) has a bleeding accelerator (54) for pressing the skin upon contact therewith in order to cause blood to flow out from the skin, said bleeding accelerator (54) being of a tapered shape that is progressively greater in diameter in a direction toward the skin.

3. An assistance device according to claim 1, further comprising:
fixing means (36, 36a, 36b) for fixing said main body (38, 38a, 110) to a blood sampling region.

4. An assistance device according to claim 1, further comprising:
biasing means (44) for biasing said lid (11, 11a, 101) in an opening direction; and
holding means (70) for holding said lid (11, 11a, 101) in a closed state.

5. An assistance device according to claim 1, wherein when said lid (11, 11a, 101) is closed, a centerline of a hole of said puncture guide (74, 112) and a centerline of a hole of said blood sampling guide (46, 116, 116a) are held in alignment with each other.

6. An assistance device according to claim 1, wherein the cross section of a hole of one of said puncture guide (74, 112) and said blood sampling guide (46, 116, 116a), which is provided on said lid (11, 11a, 101), is included within the cross section of a hole of the other of said puncture guide (74, 112) and said blood sampling guide (46, 116, 116a), which is provided on said main body (38, 38a, 110), in planar projection.

7. An assistance device according to claim 3, wherein said fixing means (36, 36a, 36b) comprises two curved arcuate ring members (86, 88, 126, 128) which form an annular shape when the ring members (86, 88, 126, 128) engage with each other, and one of the two ring members (86, 88, 126, 128) has sawtooth engaging recesses (88a, 128a) for adjusting the diameter of said fixing means (36, 36a, 36b).

8. An assistance device according to claim 3, wherein said main body (38, 38a, 110) is detachably mounted on said fixing means (36, 36a, 36b), said assistance device further comprising:
positioning means (64, 66) for positioning said main body (38, 38a, 110) with respect to said fixing means (36, 36a, 36b) in a plurality of orientations.

## Patentansprüche

1. Hilfsvorrichtung zum Punktieren der Haut mit einer Punktiervorrichtung (14, 104), die eine Punktiernadel (30, 108) hat, und zum Messen einer gewünschten Blutkomponente mit einer Messvorrichtung, an der ein Messwerkzeug (20, 106) mit einem Blutprobennehmer montiert ist, mit:
einem Hauptkörper (38, 38a, 110); und
einem Deckel (11, 11a, 101), der aufmachbar und verschließbar an den Hauptkörper (38, 38a, 110) gefügt ist,
wobei eines von dem Hauptkörper (38, 38a, 110) und dem Deckel (11, 11a, 101) eine Punktierführung (74, 112) zum Positionieren der Punktiernadel (30, 108) in Ausrichtung mit einer Punktierposition an der Haut hat, und das andere von dem Hauptkörper (38, 38a, 110) und dem Deckel (11, 11a, 101) eine Blutprobennahmeführung (46, 116, 116a) zum Positionieren des Messwerkzeugs (20, 106) in Ausrichtung mit einem Bereich der Haut hat, der durch die Punktiernadel (30, 108) punktiert wird.

2. Hilfsvorrichtung gemäß Anspruch 1, wobei der Hauptkörper (38, 38a, 110) eine Blutungsbeschleunigungseinrichtung (54) zum Drücken der Haut in Kontakt damit hat, um das Blut dazu zu bringen, von der Haut auszuströmen, wobei die Blutungsbeschleunigungseinrichtung (54) eine konische Form hat, deren Durchmesser in einer Richtung zu der Haut fortschreitend größer wird.

3. Hilfsvorrichtung gemäß Anspruch 1, ferner mit:
einem Fixierungsmittel (36, 36a, 36b) zum Fixieren des Hauptkörpers (38, 38a, 110) an einem Blutprobennahmebereich.

4. Hilfsvorrichtung gemäß Anspruch 1, ferner mit;
einem Vorspannmittel (44) zum Vorspannen des Deckels (11, 11a, 101) in einer Öffnungsrichtung; und
einem Haltemittel (70) zum Halten des Deckels (11, 11a, 101) in einem geschlossenen Zustand.

5. Hilfsvorrichtung gemäß Anspruch 1, wobei eine Mittellinie eines Lochs der Punktierführung (74, 112) und eine Mittellinie eines Lochs der Blutprobennahmeführung (46, 116, 116a) aneinander ausgerichtet gehalten werden, wenn der Deckel (11, 11a, 101) geschlossen ist.

6. Hilfsvorrichtung gemäß Anspruch 1, wobei der Querschnitt eines Lochs des einen der Punktierführung (74, 112) und der Blutprobennahmeführung (46, 116, 116a), welches an dem Deckel (11, 11a, 101) vorgesehen ist, in dem Querschnitt eines Lochs des anderen der Punktierführung (74, 112) und der Blutprobennahmeführung (46, 116, 116a), welches an dem Hauptkörper (38, 38a, 110) vorgesehen ist, in einer planaren Projektion enthalten ist.

7. Hilfsvorrichtung gemäß Anspruch 3, wobei das Fixierungsmittel (36, 36a, 36b) zwei gekrümmte, bogenförmige Ringelemente (86, 88, 126, 128) aufweist, die eine ringartige Form bilden, wenn die Ringelemente (86, 88, 126, 128) miteinander in Eingriff sind, und eines von den zwei Ringelementen (86, 88, 126, 128) Sägezahneingriffsvertiefungen (88a, 128a) zum Einstellen des Durchmessers des Fixierungsmittels (36, 36a, 36b) hat.

8. Hilfsvorrichtung gemäß Anspruch 3, wobei der Hauptkörper (38, 38a, 110) abnehmbar an dem Fixierungsmittel (36, 36a, 36b) montiert ist, wobei die Unterstützungsvorrichtung ferner Folgendes aufweist:
ein Positionierungsmittel (64, 66) zum Positionieren des Hauptkörpers (38, 38a, 110) mit Bezug auf das Fixierungsmittel (36, 36a, 36b) in einer Vielzahl von Ausrichtungen.

## Revendications

1. Dispositif d'assistance permettant de percer la peau avec un dispositif de ponction (14, 104) ayant une aiguille de ponction (30, 108), et de mesurer un composant sanguin souhaité avec un dispositif de mesure sur lequel un outil de mesure (20, 106) ayant un dispositif de prélèvement de sang est monté, comprenant :
un corps principal (38, 38a, 110) ; et
un couvercle (11, 11a, 101) relié audit corps principal (38, 38a, 110) de manière à pouvoir s'ouvrir et se fermer,
dans lequel l'un parmi ledit corps principal (38, 38a, 110) et ledit couvercle (11, 11a, 101) comprend un guide de ponction (74, 112) pour positionner ladite aiguille de ponction (30, 108) en alignement avec une position de ponction sur la peau, et l'autre parmi ledit corps principal (38, 38a, 110) et ledit couvercle (11, 11a, 101) comprend un guide de prélèvement sanguin (46, 116, 116a) pour positionner ledit outil de mesure (20, 106) en alignement avec une région de la peau qui est percée par ladite aiguille de ponction (30, 108).

2. Dispositif d'assistance selon la revendication 1, dans lequel ledit corps principal (38, 38a, 110) comporte un accélérateur de saignement (54) pour presser la peau en entrant en contact avec celle-ci afin d'amener le sang à sortir de la peau, ledit accélérateur de saignement (54) étant d'une forme conique dont le diamètre augmente progressivement dans un sens dirigé vers la peau.

3. Dispositif d'assistance selon la revendication 1, comprenant en outre :
un moyen de fixation (36, 36a, 36b) pour fixer ledit corps principal (38, 38a, 110) à une région de prélèvement de sang.

4. Dispositif d'assistance selon la revendication 1, comprenant en outre :
un moyen de sollicitation (44) pour solliciter ledit couvercle (11, 11a, 101) dans une direction d'ouverture ; et
un moyen de maintien (70) pour maintenir ledit couvercle (11, 11a, 101) dans un état fermé.

5. Dispositif d'assistance selon la revendication 1, dans lequel lorsque ledit couvercle (11, 11a, 101) est fermé, une ligne médiane d'un trou dudit guide de ponction (74, 112) et une ligne médiane d'un trou dudit guide de prélèvement de sang (46, 116, 116a) sont maintenues en alignement l'une avec l'autre.

6. Dispositif d'assistance selon la revendication 1, dans lequel la section transversale d'un trou de l'un parmi ledit guide de ponction (74, 112) et ledit guide de prélèvement de sang (46, 116, 116a), qui est prévu sur ledit couvercle (11, 11a, 101), est incluse dans la section transversale d'un trou de l'autre parmi ledit guide de ponction (74, 112) et ledit guide de prélèvement de sang (46, 116, 116a), qui est prévu sur ledit corps principal (38, 38a, 110), en projection plane.

7. Dispositif d'assistance selon la revendication 3, dans lequel ledit moyen de fixation (36, 36a, 36b) comprend deux éléments annulaires incurvés en forme d'arc (86, 88, 126, 128) qui prennent une forme annulaire lorsque les éléments annulaires (86, 88, 126, 128) s'engagent les uns avec les autres, et l'un des deux éléments annulaires (86, 88, 126, 128) comporte des évidements d'engagement en dents de scie (88a, 128a) pour ajuster le diamètre dudit moyen de fixation (36, 36a, 36b).

8. Dispositif d'assistance selon la revendication 3, dans lequel ledit corps principal (38, 38a, 110) est monté de façon amovible sur ledit moyen de fixation (36, 36a, 36b), ledit dispositif d'assistance comprenant en outre :
un moyen de positionnement (64, 66) pour positionner ledit corps principal (38, 38a, 110) par rapport audit moyen de fixation (36, 36a, 36b) dans une pluralité d'orientations.
